# EUROPEAN PATENT SPECIFICATION

(11) **EP 4 669 375 B1**
(45) Date of publication and mention of the grant of the patent: **28.10.2026**
(21) Application number: 24707308.3
(22) Date of filing: 21.02.2024
(51) Int. Cl.: A61M 1/00, A61B 17/20

(54) **CYCLIC ASPIRATION SYSTEM PRODUCING A CYCLIC ASPIRATION PRESSURE WAVEFORM USING A VACUUM PUMP AND POSITIVE PRESSURE PULSE GENERATOR MECHANISM**
ZYKLISCHE-ASPIRATIONSSYSTEM, DAS MITTELS EINER VAKUUMPUMPE UND EINES POSITIVEN DRUCKIMPULSGENERATORS EINE ZYKLISCHE DRUCKWELLENFORM ERZEUGT
SYSTÈME D'ASPIRATION CYCLIQUE PRODUISANT UNE FORME D'ONDE DE PRESSION CYCLIQUE À L'AIDE D'UNE POMPE À VIDE ET D'UN MECHANISME GÉNÉRATEUR D'IMPULSIONS DE PRESSION POSITIVE

(30) Priority: 22.02.2023 US 202363447506 P; 14.02.2024 US 202418441175
(43) Date of publication of application: 31.12.2025
(73) Proprietor: Neuravi Limited, Galway H91 K5YD (IE)
(72) Inventor: VALE, David, Galway, H91 K5YD (IE); GIBBONS, Tommy, Galway, H91 K5YD (IE); MCCARTHY, Ray, Galway, H91 K5YD (IE); MIRZA, Mahmood, Galway, H91 K5YD (IE); O'MALLEY, Thomas, Galway, H91 K5YD (IE); CARNEY, Alan, Galway, H91 K5YD (IE); GRIFFIN, Patrick, Galway, H91 K5YD (IE)
(74) Representative: Carpmaels & Ransford LLP
(86) International application number: PCT/IB2024/051643
(87) International publication number: WO 2024/176121

(56) References cited:
- WO-A1-2014/151209
- US-A- 5 511 951
- US-A1- 2019 239 910

## Description

### FIELD

The present disclosure generally relates to an aspiration system and method for use during thrombectomy procedures for the capture and removal of occlusions or clots. Specifically, the present disclosure relates to a cyclic aspiration system for the capture and removal of occlusions or clots in a vessel where the cyclic aspiration pressure waveform includes intermittent cyclic intervals of vacuum pressure (i.e., pressure below atmospheric pressure) and positive pressure (i.e., higher than vacuum pressure, possibly higher than atmospheric pressure). The cyclic aspiration system produces the cyclic aspiration waveform using a positive pressure pulse generator mechanism associated with flexible inlet tubing disposed in fluid communication between a vacuum pump and aspiration catheter, wherein the positive pressure pulse generator mechanism generates the positive pressure pulse (i.e., injection of positive pressure) by intermittently cyclically externally compressing a section along the flexible inlet tubing reducing the volume and displacing fluid collected therein thereby generating a positive pressure pulse. While no methods are claimed, the methods disclosed herein are considered useful for understanding the invention despite of not being part of it.

### BACKGROUND

Pulsatile or cyclic aspiration applies a cyclic pressure waveform of intermittent cyclic minimum/low/vacuum/aspiration pressure and maximum/peak/high pressure. During cycles under the minimum/low/vacuum/aspiration pressure the clot is drawn in the proximal direction and captured at the distal tip/end of the aspiration catheter, whereas during cycles of maximum/peak/high pressure the clot is pushed in the distal direction. When utilizing pulsatile or cyclic aspiration during the capture and removal of the clot it is desirable to maximize the cycling frequency of the cyclic pressure waveform and thus maximize clot vibration thereby optimizing aspiration performance. One key challenge in maximizing the cycling frequency is a particular response time required for mechanical actuation of each active component limiting an extent to which the cycling frequency may be increased. Complex conventional systems for maximizing cycling frequency have many active components each required to await their response times before being activated to maintain normal operation. Accordingly, in complex systems with many active components the extent to which the cycling frequency may be maximized is undesirably curtailed. Another concern is that conventional aspiration systems are prone to clogging by the captured clot.

It is therefore desirable to develop an improved cyclic aspiration system utilizing as few active components as possible with an associated maximized response time to attain maximum cycling frequency while also minimizing dampening or decay of the positive pressure wave as well as the additional benefit of reducing the overall cost of manufacture. Still further desirable wis to develop an improved cyclic aspiration system preventing, or minimizing, risk of clogging.

US2019239910A1 describes a system for removing occlusive clot from a blood vessel comprises a catheter and an apparatus for generating a pulsatile vacuum force to pulse the pressure gradient at a distal end of the catheter. The pulse generator may be integral with or separate from the vacuum pump. The pulse generator may be applied to a flexible tubing between the vacuum pump and the proximal end of the catheter.

### SUMMARY

An aspect of the present disclosure relates to a pulsatile or cyclic aspiration system producing a cyclic aspiration pressure waveform of intermittent cyclic intervals of vacuum pressure below atmospheric pressure and positive pressure higher than vacuum pressure (higher than vacuum pressure, possibly higher than atmospheric pressure) using as few active components as possible with an associated maximized response time to attain maximum cycling frequency while also minimizing dampening or decay of the positive pressure wave as well as the additional benefit of reducing the overall cost of manufacture.

Another aspect of the present disclosure is directed to a cyclic aspiration system producing a cyclic aspiration pressure waveform using a vacuum pump connected in fluid communication with an aspiration catheter via flexible inlet tubing and a positive pressure pulse generator mechanism imposing an external force compressing a section of the flexible inlet tubing reducing the internal volume displacing fluid collected therein thereby creating the positive pressure pulse (e.g., injection of positive pressure).

An aspect of the present disclosure is directed to a cyclic aspiration system producing a cyclic aspiration pressure waveform of intermittent cyclic intervals of vacuum pressure (i.e., pressure below atmospheric pressure) and positive pressure (i.e., higher than vacuum pressure, possibly higher than atmospheric pressure) in which the inexpensive flexible inlet tubing contaminated by blood is discardable after a single use, whereas more expensive components imposing the external force compressing the flexible inlet tubing are not contaminated by blood and hence reusable.

While yet another aspect of the present disclosure is directed to a cyclic aspiration system producing a cyclic aspiration pressure waveform using a vacuum pump and a positive pressure pulse generator by applying an external force compressing a section of flexible inlet tubing connected in fluid communication between the vacuum pump and hub of the aspiration catheter, while forcibly restoring the flexible inlet tubing to the non-compressed state to hasten recovery time while maximizing attainable cycling frequency.

### BRIEF DESCRIPTION OF THE DRAWINGS

The above and further aspects of the present disclosure are further discussed with reference to the following description in conjunction with the accompanying drawings, in which like numerals indicate like structural elements and features in various figures. The drawings are not necessarily to scale, emphasis instead being placed upon illustrating principles of the present disclosure. The figures depict one or more implementations of the devices, by way of example only, not by way of limitation.

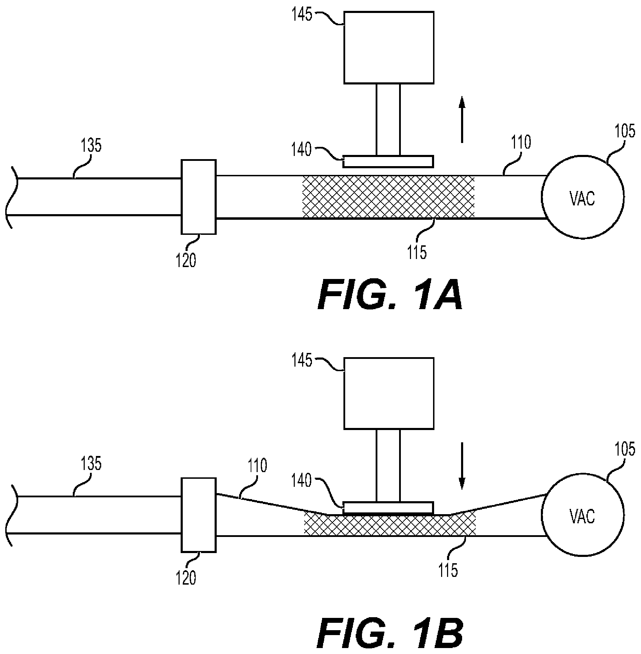

Figure 1A is an example cyclic aspiration system in accordance with the present disclosure in which the positive pressure pulse generator mechanism is a displaceable plunger arranged externally along a section of the flexible inlet tubing (i.e., vacuum line) disposed between the vacuum pump and hub of the aspiration catheter; wherein the plunger is depicted in a retracted (i.e., withdrawn) state with the flexible inlet tubing in a non-compressed state;
Figure 1B is the example cyclic aspiration system of Figure 1A wherein the plunger is depicted in an advanced (i.e., extended) state compressing the flexible inlet tubing (i.e., compressed state);

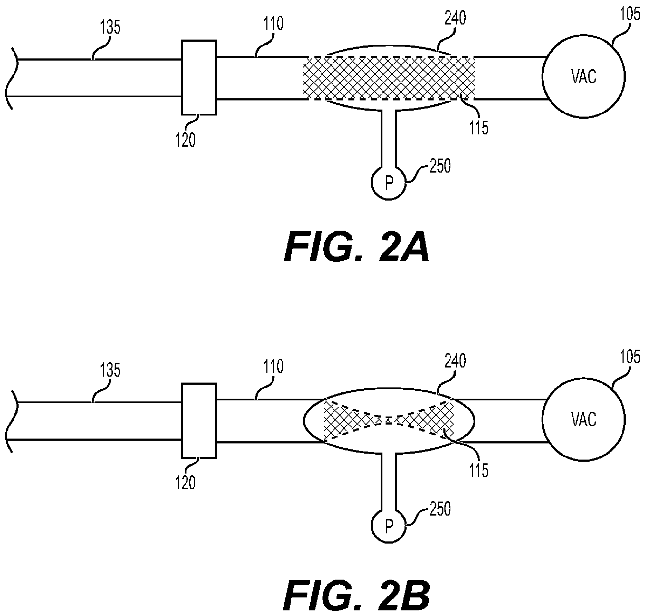

Figure 2A is another example cyclic aspiration system in accordance with the present disclosure in which the positive pulse generator mechanism is a pressurizable bladder arranged externally along a section of the flexible inlet tubing (i.e., vacuum line) disposed between the vacuum pump and hub of the aspiration catheter; wherein the bladder is depicted in a non-pressurized state with the flexible inlet tubing in a non-compressed state;
Figure 2B is the example cyclic aspiration system of Figure 2A wherein the bladder is depicted in a pressurized state radially constricting or compressing the inlet tubing (i.e., compressed state);

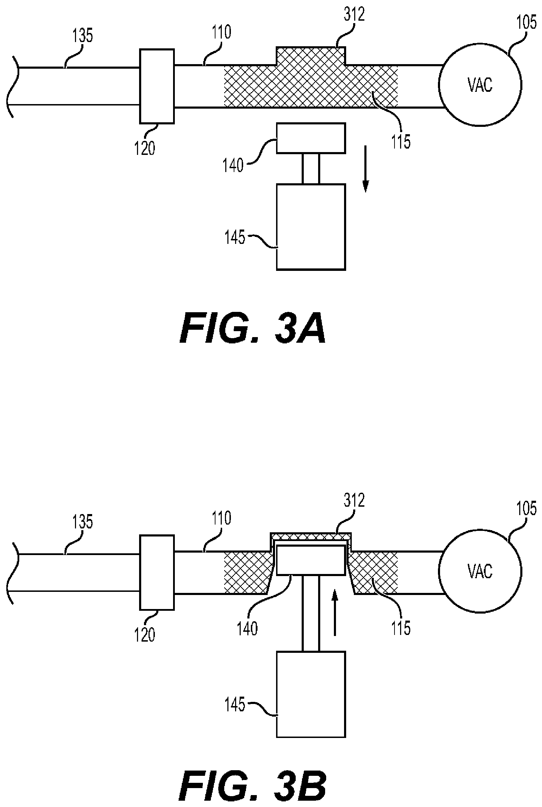

Figure 3A is still another example cyclic aspiration system in accordance with the present disclosure in which the positive pulse generator mechanism is a displaceable plunger arranged externally along a section of the flexible inlet tubing (i.e., vacuum line) disposed between the vacuum pump and hub of the aspiration catheter including a recess for receiving therein the plunger while in an advanced state; wherein the plunger is depicted in a retracted (i.e., withdrawn) state with the flexible inlet tubing in a non-compressed state;
Figure 3B is the example cyclic aspiration system of Figure 3A wherein the plunger is depicted in an advanced (i.e., extended) state seated in the recess while compressing the inlet tubing (i.e., compressed state);

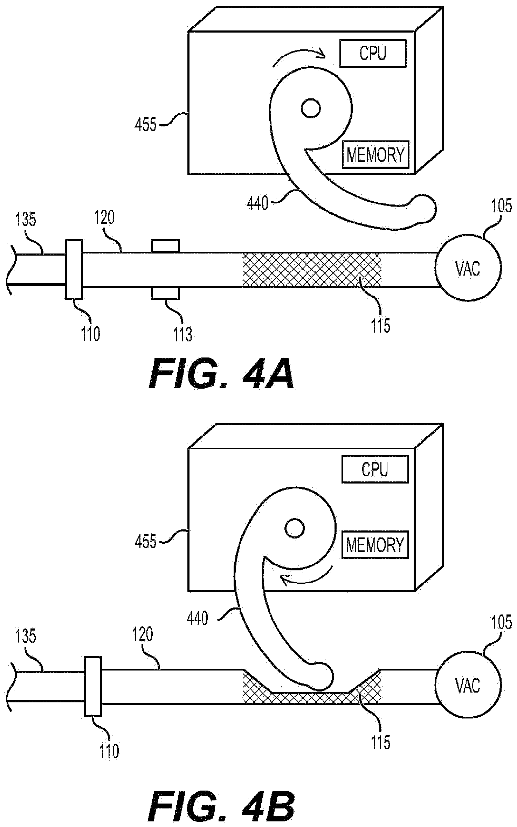

Figure 4A is yet still another example cyclic aspiration system in accordance with the present disclosure in which the positive pulse generator mechanism is a rotatable arm arranged externally along a section of the inlet tubing (i.e., vacuum line) disposed between the vacuum pump and hub of the aspiration catheter; wherein the rotatable arm is depicted in a retracted (i.e., withdrawn) state with the flexible inlet tubing in a non-compressed state;
Figure 4B is the example cyclic aspiration system of Figure 4A wherein the rotatable arm is depicted in an advanced (i.e., extended) state compressing the inlet tubing (i.e., compressed state) and displacing volume within the tubing towards the hub of the aspiration catheter;

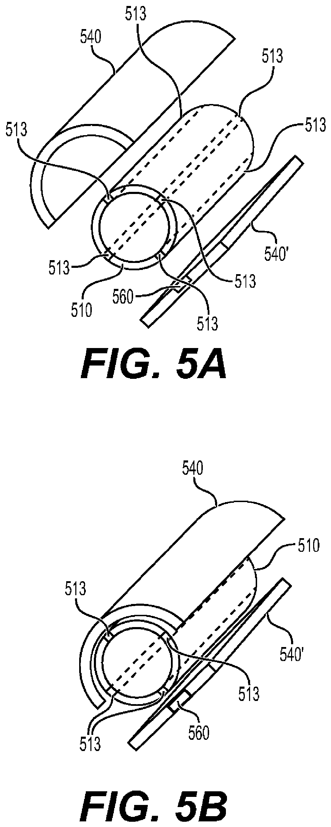

Figure 5A is a perspective exploded view of an example positive pulse injection mechanism including a pair of electromagnets (a concave electromagnet and a planar base plate electromagnet) arranged externally along a section of the flexible inlet tubing (i.e., vacuum line) disposed between the vacuum pump and hub of the aspiration catheter where the flexible inlet tubing contains conductive strips embedded in the tubing wall and the base plate electromagnet also includes a permanent magnet;
Figure 5B is a perspective view of the positive pressure pulse generator mechanism of Figure 5A assembled along the section of the flexible inlet tubing;

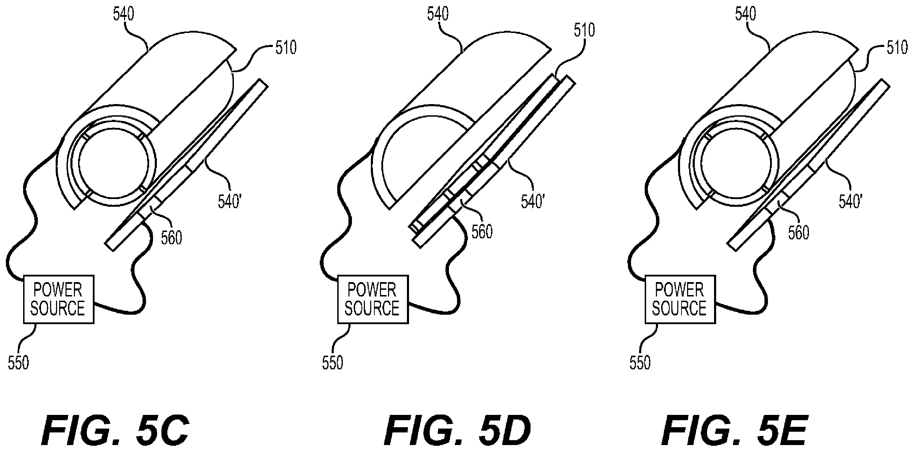

Figure 5C is a perspective view of the positive pressure pulse generator mechanism of Figure 5B depicting the flexible inlet tubing in a non-compressed state drawn to the concave contacting surface of the first electromagnet when it is energized;
Figure 5D is a perspective view of the positive pulse generator mechanism of Figure 5B depicting the flexible inlet tubing in a compressed state drawn to a planar contacting surface of the second electromagnet when it is energized ("ON"), and the first concave electromagnet is not energized ("OFF");
Figure 5E is a perspective view of the positive pressure pulse generator mechanism of Figure 5B illustrating hastened (i.e., forced) recovery to its non-compressed state the compressed flexible inlet tubing held in place via a permanent magnet while simultaneously being drawn to the concave contacting surface of the energized concave electromagnet;

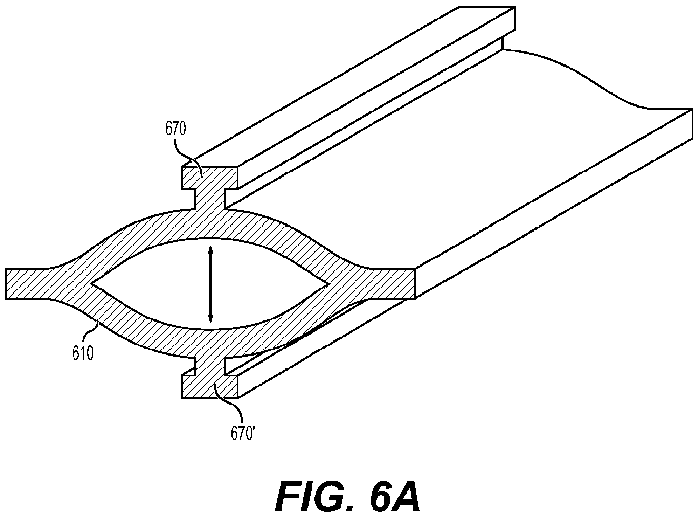

Figure 6A is a perspective view of an example extruded, cast, or molded flexible inlet tubing having a non-circular radial cross-section imposing a radial resistance feature (e.g. fins) preventing collapse under vacuum pressure and the gripping elements (e.g., rails) hasten (i.e., force) recovery or return of the flexible inlet tubing to a non-compressed state when the external force is withdrawn;

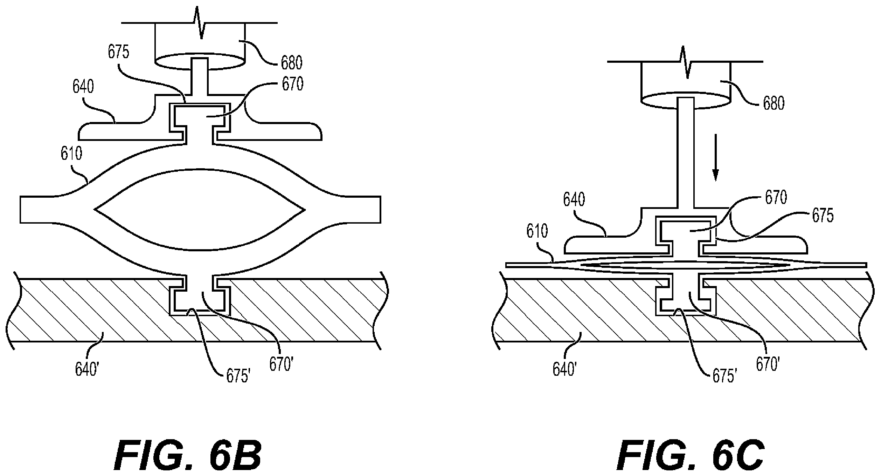

Figure 6B depicts an example positive pressure pulse generator mechanism for the extruded or cast flexible inlet tubing in 6A as a displaceable compression plate and stationary base each having slots for slidably securing therein respective gripping elements (e.g., rails) of the extruded flexible inlet tubing shown in a non-compressed state, thus allowing unrestricted passage through the flexible inlet tubing to the vacuum pump while preventing collapse of the flexible inlet tubing under vacuum;
Figure 6C depicts the example positive pressure pulse generator mechanism of Figure 6B with the compression plate shown in an advanced (e.g., extended) state compressing the extruded or cast flexible inlet tubing (i.e., compressed state), thus restricting passage to the vacuum pump and simultaneously displacing fluid collected within the flexible inlet tubing towards the catheter hub generating the positive pressure pulse;

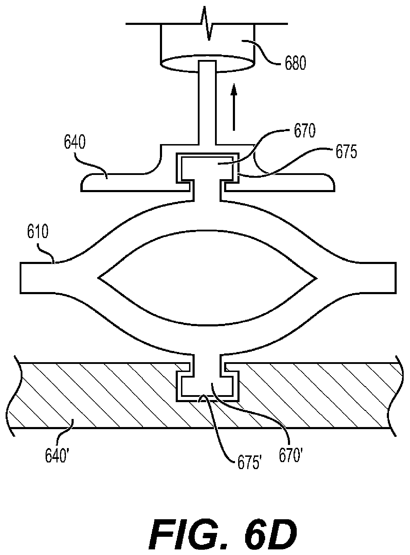

Figure 6D depicts the example positive pressure pulse generator mechanism of Figure 6B illustrating the hastened (i.e., forced) recovery of the extruded flexible inlet tubing to a restored (i.e., non-compressed) state during retraction of the compression plate with the extruded flexible inlet tubing secured thereto and radial resistance exhibited by the extruded flexible inlet tubing thus allowing unrestricted access to the vacuum pump through the flexible inlet tubing again, and supplying radial force (through the radial resistance elements (e.g., fins) to the flexible inlet tubing to prevent collapse while under vacuum;

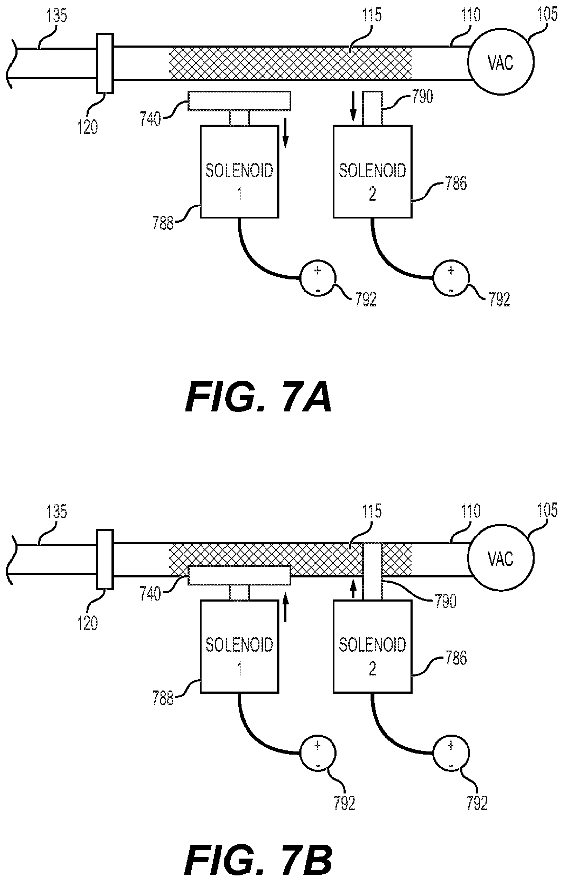

Figure 7A is still yet another example cyclic aspiration system in accordance with the present disclosure including a vacuum pump in fluid communication with a hub of an aspiration catheter via a flexible inlet tubing along a section of which is arranged a positive pressure pulse generator mechanism (e.g., a displaceable plunger) and proximally thereto (on the vacuum pump end of the tubing) a separate independently displaceable gating mechanism (e.g. a pinch-valve); wherein the displaceable gating mechanism is in a retracted (i.e., withdrawn or open) state allowing unrestricted passage therethrough of the vacuum pressure and the plunger is depicted in a retracted (i.e., withdrawn) state with the flexible inlet tubing in a non-compressed state;
Figure 7B is the example cyclic aspiration system of Figure 7A wherein the the displaceable gating mechanism is in an advanced (i.e., extended) state restricting passage therethrough of the vacuum pressure and the plunger is depicted in an advanced (i.e., extended) state partially or fully compressing the flexible inlet tubing (i.e., compressed state) to displace the fluid collected therein in the direction of the catheter hub to generating a positive pressure pulse, while preventing the fluid from being displaced into the vacuum pump by the displaceable gating mechanism;

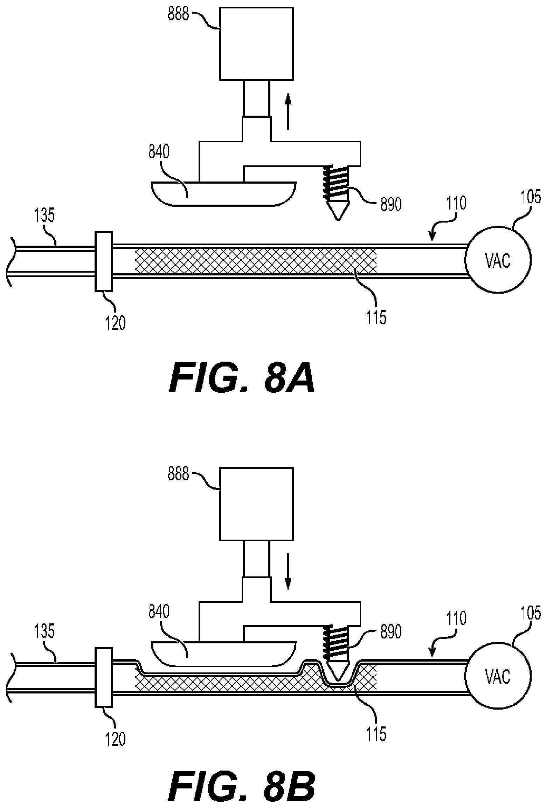

Figure 8A is a modified example of the cyclic aspiration system of Figure 7A in which the positive pressure pulse generator mechanism (e.g., a displaceable plunger head) and displaceable gating mechanism (e.g., spring-loaded pin head) arranged proximally (on the side of the vacuum pump) thereto are integrated into a single mechanism simultaneously displaceable via a single actuator; wherein the gating mechanism is extends further towards the flexible inlet tubing relative to that of the pulse generator mechanism to ensure compression of the flexible inlet tubing first by the gating mechanism; wherein the displaceable plunger head is depicted in a retracted (i.e., withdrawn) state with the flexible inlet tubing in a non-compressed state and the spring-loaded pin head is in a retracted (i.e., withdrawn) state allowing unrestricted passage therethrough of the vacuum pressure;
Figure 8B is the example cyclic aspiration system of Figure 8A wherein the the spring-loaded pin head is in an advanced (i.e., extended) state restricting passage therethrough of the vacuum pressure and the displaceable plunger head is depicted in an advanced (i.e., extended) state partially or fully compressing the flexible inlet tubing (i.e., compressed state) thus displacing the fluid in the line and generating the positive pressure pulse in the direction of the catheter hub while preventing displacement of the fluid into the vacuum pump by the pin head;

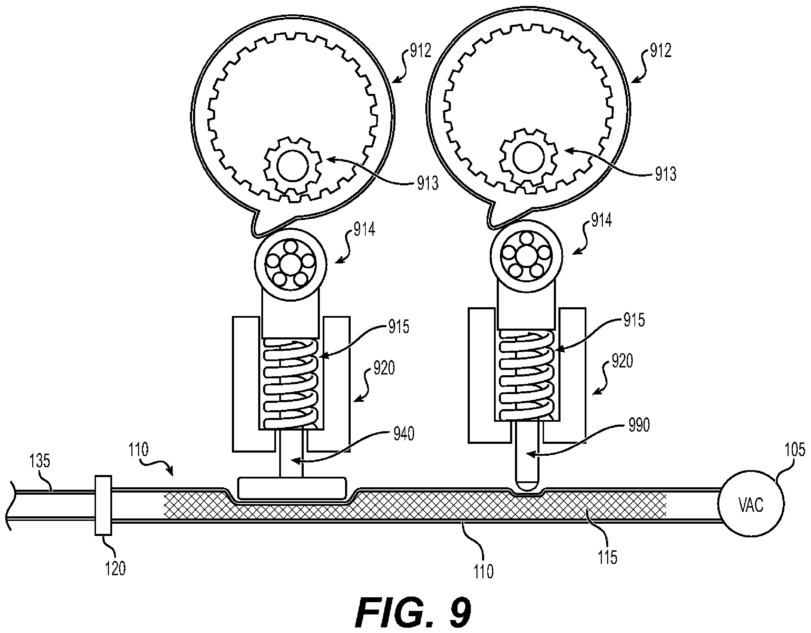

Figure 9 is still yet another example cyclic aspiration system in accordance with the present disclosure including a vacuum pump in fluid communication with a hub of an aspiration catheter via a flexible inlet tubing along a section of which is arranged a positive pressure pulse generator mechanism (e.g., a reciprocating plunger) and proximally thereto a separate independently actuated reciprocating valve mechanism (e.g., a reciprocating pin); wherein both the plunger mechanism and the pin mechanism are each driven by a cam with an internal gear, which is connected to motor, wherein the motor-driven cam contacts a roller-bearing which causes the mechanisms to advance into the flexible inlet tubing; depicting the reciprocating pin in an advanced (i.e., extended) state restricting passage therethrough of the vacuum pressure while the reciprocating plunger is in an advanced (i.e., extended) state compressing the flexible inlet tubing displacing the fluid therein thereby generating the positive pressure pulse in the direction of the catheter hub while preventing displacement of the fluid into the vacuum pump by the pin;

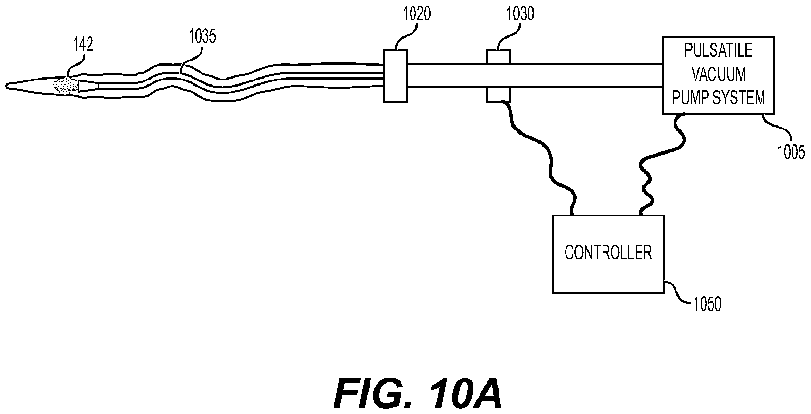

Figure 10A is a diagram depicting inlet tubing connecting in fluid communication a proximal hub attached to an aspiration catheter and a pulsatile vacuum pump for producing a cyclic aspiration pressure waveform including intermittent intervals of vacuum pressure (i.e., below atmospheric pressure) and positive pressure (i.e., higher than vacuum pressure, possibly higher than atmospheric pressure); wherein adjustment is made to at least one parameter (e.g., amplitude of vacuum pressure, amplitude of positive pressure pulse, or cycling frequency) of the cyclic aspiration pressure waveform associated with the pulsatile vacuum pump based on a determination of percentage fibrin content of a clot captured at the distal tip of the aspiration catheter in response to a cyclic pressure waveform detected over time by pressure sensor(s);

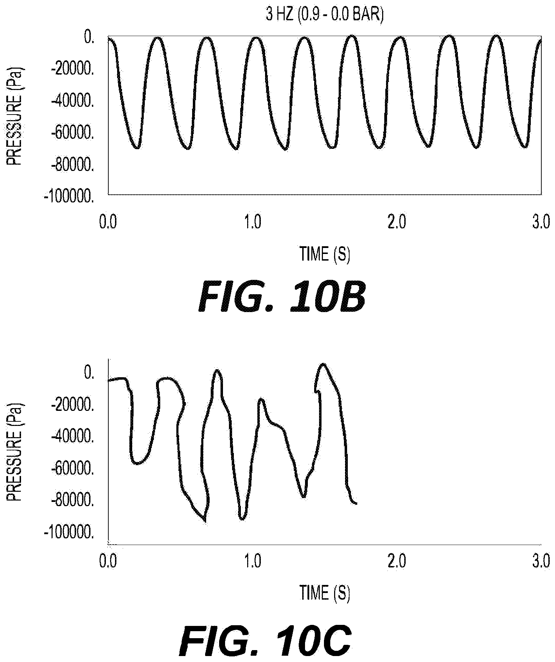

Figure 10B is a graphical representation of an exemplary substantially consistent repeatable or regular detected pressure waveform over time for a captured firm clot having a high fibrin content.
Figure 10C is a graphical representation of an exemplary substantially inconsistently repeatable or irregular detected pressure waveform over time for a captured friable clot having a low fibrin content.

### DETAILED DESCRIPTION

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

As used herein, the terms "component," "module," "system," "server," "processor," "memory," and the like are intended to include one or more computer-related units, such as but not limited to hardware, firmware, a combination of hardware and software, software, or software in execution. For example, a component may be, but is not limited to being, a process running on a processor, an object, an executable, a thread of execution, a program, and/or a computer. By way of illustration, both an application running on a computing device and the computing device can be a component. One or more components can reside within a process and/or thread of execution and a component may be localized on one computer and/or distributed between two or more computers. In addition, these components can execute from various computer readable media having various data structures stored thereon. The components may communicate by way of local and/or remote processes such as in accordance with a signal having one or more data packets, such as data from one component interacting with another component in a local system, distributed system, and/or across a network such as the Internet with other systems by way of the signal. Computer readable medium can be non-transitory. Non-transitory computer-readable media include, but are not limited to, random access memory (RAM), read-only memory (ROM), electronically erasable programmable ROM (EEPROM), flash memory or other memory technology, compact disc ROM (CD-ROM), digital versatile disks (DVD) or other optical storage, magnetic cassettes, magnetic tape, magnetic disk storage or other magnetic storage devices, or any other tangible, physical medium which can be used to store computer readable instructions and/or data.

As used herein, the term "computing system" is intended to include stand-alone machines or devices and/or a combination of machines, components, modules, systems, servers, processors, memory, detectors, user interfaces, computing device interfaces, network interfaces, hardware elements, software elements, firmware elements, and other computer-related units. By way of example, but not limitation, a computing system can include one or more of a general-purpose computer, a special-purpose computer, a processor, a portable electronic device, a portable electronic medical instrument, a stationary or semi-stationary electronic medical instrument, or other electronic data processing apparatus.

As used herein, the terms "tubular" and "tube" are to be construed broadly and are not limited to a structure that is a right cylinder or strictly circumferential in cross-section or of a uniform cross-section throughout its length. For example, a tubular structure or system is generally illustrated as a substantially right cylindrical structure. However, the tubular system may have a tapered or curved outer surface.

The present disclosure relates to a cyclic aspiration system for producing a cyclic aspiration pressure waveform using a vacuum pump and a positive pressure pulse generator mechanism disposed externally along a section of flexible (i.e., compressible) inlet tubing (i.e., vacuum line) connected in fluid communication between the vacuum pump and proximal hub of the aspiration catheter. The cyclic aspiration pressure waveform of intermittent cyclic intervals of vacuum pressure (i.e., below atmospheric pressure) and positive pressure (i.e., higher than the vacuum pressure, possibly higher than atmospheric pressure) is generated in accordance with the present disclosure via the positive pressure pulse generator mechanism intermittently cyclically applying an external force compressing the flexible inlet tubing reducing the volume and displacing the fluid collectable therein thereby creating a positive pressure pulse (i.e., injection of positive pressure) negating the vacuum pressure. Generation of the cyclic aspiration pressure waveform utilizing internal solenoids would undesirably become contaminated by aspirated blood. The positive pressure pulse generator mechanism in accordance with the present disclosure advantageously is arranged externally of the flexible inlet tubing (i.e., vacuum line), not contaminated by blood, and therefore reusable; whereas the flexible inlet tubing is inexpensive, contaminated by blood, and discardable after a single use thereby preventing clogging.

Numerous factors must be considered in maximizing the oscillation or cycling frequency when varying pressure (e.g., vacuum pressure and positive pressure pulse) to produce the cyclic aspiration pressure waveform. In accordance with the present disclosure, the positive pressure is injected or generated in the flexible inlet tubing proximal to the vacuum pump. Another factor addressed by the positive pressure pulse generator mechanism in accordance with the present disclosure is maximizing the oscillation or cycling frequency when creating the positive pressure pulse via the compression of the flexible inlet tubing (i.e., vacuum line). The restoration or recovery time for the flexible inlet tubing to return to its non-compressed state naturally on its own (i.e., unforced, unaided, or unassisted) following withdrawal of an external compressive force is too slow resulting in unacceptably low cycling frequencies. Maximized cycling frequency (preferably in a range between approximately 1Hz and approximately 20Hz) is realized in accordance with the present disclosure by forcing (i.e., hastening) return of the compressed flexible inlet tubing to its non-compressed state (i.e., minimizing restoration or recovery time) upon withdrawal of the external compressive force.

Several non-limiting examples are illustrated and described herein of cyclic aspiration systems producing a cyclic aspiration pressure waveform of intermittent cyclic intervals of vacuum pressure (i.e., pressure below atmospheric pressure) and positive pressure (i.e., higher than vacuum pressure, possibly higher than atmospheric pressure) using a positive pressure pulse injection mechanism imposing an external force compressing a section of flexible inlet tubing in fluid communication between the vacuum pump and hub attached to an aspiration catheter. Return of the compressed flexible inlet tubing to its non-compressed state upon withdrawal of the external compressive force in each of the examples described herein is forced, assisted, or hastened is some manner thereby minimizing recovery time while maximizing cycling frequency.

Figure 1A is a diagram of an exemplary cyclic aspiration system 100 in accordance with the present disclosure in which a proximal hub 120 of an aspiration catheter 135 is connected in fluid communication to a vacuum pump 105 via flexible (i.e., compressible) inlet tubing 110 (i.e., vacuum inlet line). For instance, the inlet tubing 110 may be made of an elastomer, silicone, rubber, or latex. Along a section of the flexible inlet tubing 110 distally of the vacuum pump 105 is a positive pressure pulse generator mechanism imposing an externally compressive force compressing or constricting a section of the flexible inlet tubing 110 reducing the volume thereby displacing fluid collectable therein creating a positive pressure pulse (i.e., injection of positive pressure) negating the vacuum pressure. In the example of Figure 1A the positive pressure pulse generator mechanism is a displaceable plunger 140 moved by a linear displacement mechanism 145 (e.g., a linear actuator, solenoid, reciprocating motor, cam, rotating motor, etc.) to be intermittently cyclically displaceable between a non-deployed (i.e., retracted) state and a deployed (i.e., advanced or extended) state, as shown in Figures 1A & 1B, respectively. Both the plunger 140 and linear displacement mechanism 145 are arranged externally of the flexible inlet tubing 110. To maximize the cycling or oscillating frequency switching between vacuum pressure and positive pressure, a radially self-expanding restoring structure 115 (e.g., braid, skeleton, spring, or cage) is disposed within the lumen of the flexible inlet tubing 110 coinciding with the section compressed by the positive pressure pulse generator mechanism. The radially self-expanding restoring structure 115 may be any radially self-expandable restorative mechanical structure that returns to an original, natural, or default (i.e., non-compressed) shape upon withdrawal of an external compressive force. Specifically, the radially self-expanding restoring structure 115 minimizes restoration or recovery time for the compressed flexible inlet tubing 110 to return to its non-compressed shape upon the removal or withdrawal of the external compressive force (e.g., retraction of the plunger 140), as shown in Figure 1A. In addition, the radially self-expanding structure also resists or prevents collapse of the flexible inlet tubing under vacuum.

In operation, once the aspiration catheter 135 is delivered through the vasculature to the target site on a proximal side/face of the clot, the vacuum pump 105 generates the vacuum pressure received in the aspiration catheter 135 via the flexible inlet tubing 110. Linear displacement member 145 intermittently cyclically displaces the plunger 140 compressing or constricting the flexible inlet tubing 110 reducing the volume displacing the fluid collected therein thereby generating a positive pressure pulse (i.e., injection of positive pressure) negating the vacuum pressure (Figure 1B). Upon withdrawal of the external force on the flexible inlet tubing 110 (e.g., retraction of the plunger 140) (Figure 1A), the radially self-expanding restoring structure 115 forces or hastens return of the flexible inlet tubing 110 to its original (i.e., non-compressed) shape thereby minimizing recovery time and maximizing cycling frequency. In addition, the radially self-expanding restoring structure 115 also resists or prevents collapse of the flexible inlet tubing 110 under vacuum. Both the plunger 140 and linear displacement member 145 are arranged externally of the flexible inlet tubing 110.

Figure 2A is another example of the positive pressure pulse mechanism in accordance with the present disclosure as a pressurized bladder 240 filled with a liquid and/or gas disposed along a section of the flexible inlet tubing 110. In lieu of a motor, a supplemental pump 250 oscillates imposing a pressure (P) on the bladder 240 constricting or squeezing a section of the flexible inlet tubing 110. The exerted pressure (P) transitions the bladder 240 from a non-pressurized state (i.e., non-compression of the flexible inlet tubing 110) to a pressurized state (i.e., compressing the flexible inlet tubing 110), as depicted in Figures 2A & 2B, respectively (the inner wall of the flexible inlet tubing 110 depicted by the dashed line). To maximize the cycling or oscillating frequency switching between vacuum pressure and positive pressure, the radially self-expanding restoring structure 115 forces or assists return of the compressed flexible inlet tubing 110 to its non-compressed state when no longer constricted by the bladder 240 (i.e., minimizing recovery time). In addition, the radially self-expanding restoring structure 115 also resists or prevents collapse of the flexible inlet tubing 110 under vacuum.

In operation, once the aspiration catheter 135 is delivered through the vasculature to the target site on a proximal side/face of the clot, the vacuum pump 105 is activated generating the vacuum pressure received in the aspiration catheter 135 via the flexible inlet tubing 110 while the bladder 240 is the non-pressurized state (i.e., non-compression of the flexible inlet tubing 110). In response to an externally applied force generated by the supplemental pump (P) 250, the bladder 240 transitions to the pressurized state constricting or squeezing the flexible inlet tubing 110 (i.e., compressed state) reducing the volume and displacing the fluid collected therein thereby creating a positive pressure pulse (i.e., injection of positive pressure) negating the vacuum pressure. Upon withdrawal of the external force from the supplemental pump (P) 250 on the bladder 240 (Figure 2A), the radially self-expanding restoring structure 115 forces or hastens restoration of the flexible inlet tubing 110 to its non-compressed shape or state minimizing recovery time while maximizing cycling frequency (Figure 2B). In addition, the radially self-expanding restoring structure 115 also resists or prevents collapse of the flexible inlet tubing 110 under vacuum.

The previously described positive pressure pulse generator mechanisms (Figures 1A-1B) arranged externally of the flexible inlet tubing 110 pose a potential risk of hang up of the clot at the pinch point (i.e., tapered region of the flexible inlet tubing 110 distally of the compressed section). The modified positive pressure pulse generator mechanism in Figures 3A & 3B addresses this issue by configuring the plunger 140 in the flexible inlet tubing 110 (i.e., vacuum line). Referring to Figure 3A, the profile of the flexible inlet tubing 310 (i.e., vacuum line) is modified to include a recess 312 conforming, matching, or complementary in shape to seat the plunger 140 therein. Accordingly, when displaced (i.e., advanced) by the linear displacement mechanism 145 (e.g., linear actuator, solenoid, reciprocating motor, cam, rotating to reciprocating mechanism, etc.), the plunger 140 is seated within the recess 312 preventing or minimizing possible hang-up of the clot by eliminating the pinch point. In operation, once the aspiration catheter 135 is delivered through the vasculature to the target site on a proximal side/face of the clot, the vacuum pump 105 generates the vacuum pressure received in the aspiration catheter 135 via the inlet tubing 110. Linear displacement mechanism 145 intermittently cyclically advances the plunger 140 to a position seated in the recess 312 compressing or constricting the flexible inlet tubing 110 reducing the volume and displacing the collected fluid therein thereby creating a positive pressure pulse (i.e., injection of positive pressure) negating the vacuum pressure. Upon withdrawal of the external force on the plunger 140 (Figure 3A), the radially self-expanding restoring structure 115 forces or hastens return of the flexible inlet tubing 110 to its original, natural, default (i.e., non-compressed) shape or state minimizing recovery time while maximizing cycling frequency. In addition, the radially self-expanding restoring structure 115 also resists or prevents collapse of the flexible inlet tubing 110 under vacuum.

In still another example the positive pressure pulse generator mechanism is a curved arm (e.g., cam arm) 440, as shown in Figures 4A & 4B, electronically operated via a motor 455 including a programmable controller (e.g., processor or central processing unit (CPU)) and corresponding programmable memory device (e.g., RAM, ROM, EPROM, etc.) for storing therein instructions to intermittently control frequency and/or extent or rotation of the arm 440 between a non-deployed (i.e., retracted) state (Figure 4A) and a deployed (i.e., advanced or extended) state (Figure 4B). In operation, once the aspiration catheter 135 is delivered through the vasculature to the target site on a proximal side/face of the clot, the vacuum pump 105 generates the vacuum pressure received in the aspiration catheter 135 via the flexible inlet tubing 110. Motor 455 controls rotation of the arm 440 intermittently cyclically transitioning between the retracted state (Figure 4A) and the advanced (i.e., extended) state (Figure 4B) compressing or constricting the flexible inlet tubing 110 reducing the volume and displacing the fluid collected therein thereby creating a positive pressure pulse (i.e., injection of positive pressure). Upon withdrawal of the compressive force on the flexible inlet tubing 110 (e.g., retraction of the arm 440) (Figure 4A), the radially self-expanding restoring structure 115 forces or hastens return of the flexible inlet tubing 110 to its original, natural, or default (i.e., non-compressed) shape or state thereby minimizing recovery time while maximizing cycling frequency. In addition, the radially self-expanding restoring structure 115 also resists or prevents collapse of the flexible inlet tubing 110 under vacuum. A pressure sensor 113 associated with the flexible inlet tubing 110 distally of the positive pressure pulse generator mechanism (e.g., rotatable arm 440) to control, adjust, or vary the amplitude of the positive pressure pulse generated by controlling (via the processor associated with the motor 455) the extent of rotation of the arm 440 and thus the extent of compression of the flexible inlet tubing 110. Specifically, a maximum positive pressure pulse is generated when the arm 440 is rotated to a maximum extended position (i.e., fully advanced position) resulting in maximum, full, or complete compression of the flexible inlet tubing 110, whereas only partial compression of the flexible inlet tubing 110 will generate a lower amplitude positive pressure pulse. Optionally, the distance separation between the center of the arm 440 relative to the flexible inlet tubing 110 may be varied using a linear displacement mechanism (e.g., a linear actuator) to adjust the amplitude of the generated positive pressure pulse. Raising the height of the arm 440 lessening or only partially compressing the flexible inlet tubing 110 producing a lower amplitude positive pressure pulse, whereas by lowering the height of the arm 440 the flexible inlet tubing 110 is more or possibly fully compressed generating a positive pressure pulse of greater amplitude.

While still another example of the positive pressure pulse generator mechanism in accordance with the present disclosure utilizes a plurality of electromagnets arranged externally of the flexible inlet tubing intermittently cyclically compressing or constricting the flexible inlet tubing. In Figures 5A-5E the flexible inlet tubing 510 is electrically conductive. For example, the flexible inlet tubing 510 may include a plurality of conductive metal elements 513 (e.g., conductive metal wires or strips) associated therewith (e.g., disposed in the lumen, co-extruded, and/or embedded in the wall thereof). Flexible inlet tubing 510 preferably has four radially equidistantly spaced conductive metal strips 513 extending in an axial or longitudinal direction. A first electromagnet 540 having a concave contacting surface and a second electromagnet 540' having a planar (i.e., flat) contacting surface with the electrically conductive flexible inlet tubing 510 disposed therebetween. Preferably, the concave contacting surface of the first electromagnet 540 and flexible inlet tubing 510 are substantially equal in diameter.

In operation once the aspiration catheter is delivered through the vessel to the target site on a proximal side/face of the targeted clot the vacuum pump is activated. The cyclic aspiration pressure waveform is generated by intermittently energizing via a power source 550 the first electromagnet 540 or the second electromagnet 540'. Specifically, while the second electromagnet 540' remains de-energized (i.e., switched "OFF"), the first electromagnet 540 is energized (i.e., switched "ON") with the flexible inlet tubing 110 seated against the concave contacting surface (Figure 5C) allowing unrestricted or maximum passage of the vacuum pressure therethrough aspirating the clot at the distal tip of the aspiration catheter (i.e., vacuum pressure interval). Cycling to an interval of positive pressure is achieved by de-energizing (i.e., switching "OFF") the first electromagnet 540 while energizing (i.e., switching "ON") the second electromagnet 540' drawn to its planar contacting surface thereby at least partially (possibly fully) collapsing, compressing, or flattening the flexible inlet tubing 110 (Figure 5D). In the example illustrated in Figure 5D, inlet tubing 510 is depicted completely, fully, or totally collapsed or flattened onto itself. However, partial collapse, compression, or flattening of the flexible inlet tubing 510 is possible by varying the energy level to the second electromagnet 540' thereby controlling the amplitude of the positive pressure pulse generated. As with the previously described examples, compressing the flexible inlet tubing reduces the volume and displaces the fluid collected therein thereby generating the positive pressure pulse (i.e., injection of positive pressure). This cycling repeats by de-energizing (i.e., switching "OFF") the second electromagnet 540' while energizing (i.e., switching "ON") the first electromagnet 540 (Figure 5E). When energized, the first electromagnet 540 draws the flexible inlet tubing 110 thereto engaging with the concave contacting surface forcing or assisting in return of this "upper" section to its non-compressed shape or state. Simultaneously therewith, forced or hastened return of the "lower" section of the flexible inlet tubing 110 to its non-compressed shape or state is provided by a permanent magnet 560 extending in an axial or longitudinal direction along the second electromagnet 540' retaining or holding the flexible inlet tubing 110 in place. Alternatively, the permanent magnet may be replaced by a mechanical feature or component holding or retaining the flexible inlet tubing to the lower base plate (e.g., rails as in Figures 6A-6D, a clip, a clamp, or any other mechanical holding device). This forced or hastened return of respective "upper" and "lower" sections of the compressed flexible inlet tubing 110 to its non-compressed state or shape minimizes recovery time while maximizing cycling frequency. In addition, the magnets 540, 540, 560 also resists or prevents collapse of the flexible inlet tubing 110 under vacuum.

If lieu of the magnets (Figures 5A-5E), restoration of the compressed flexible inlet tubing to its non-compressed state or shape may be forced or hastened using mechanical components (e.g., gripping elements) thereby minimizing recovery time while maximizing cyclic frequency. In the example depicted in Figures 6A-6C, the flexible inlet tubing 610 is at least partially compressed, collapsed, constricted, or flattened between a displaceable compression plate 640 and stationary base 640'. Gripping elements along the flexible inlet tubing 610 are receivable within retaining elements associated with each of the displaceable compression plate 640 and stationary base 640'. In the example in Figures 6A-6D, the flexible inlet tubing 610 has rails 670, 670' (e.g., T-shape rails) extending in an axial or longitudinal direction slidable within slots 675', 657' defined in each of the displaceable compression plate 640 and stationary base 640' securing the flexible inlet tubing 610 in place therebetween. Securement of the flexible inlet tubing 610 to each of the displaceable compression plate 640 and stationary base 640' forces or hastens return of the compressed flexible inlet tubing 610 to a non-compressed state or shape during retraction of the compression plate 640 relative to the stationary base 640' thereby minimizing recovery time while maximizing cycling frequency. To further assist in hastening recovery time while maximizing cycling frequency, preferably, the flexible inlet tubing 610 may be extruded, cast, or molded into a non-circular shape with parallel tapered longitudinal sides (e.g., "lip-shaped") providing radial resistance. In addition, this geometry resists the transverse collapse of the flexible inlet tubing under vacuum when the compression plate 640 is retracted and the flexible inlet tubing 610 is open to the vacuum pump. Although not essential, the non-circular shape also fosters forming of a complete seal of the flexible inlet tubing (i.e., vacuum line) when pressed or squeezed between the compression elements (e.g., plunger 640 and base 640') if full, maximum, or complete compression of the flexible inlet tubing is desired. Prior to advancement of the compression plate 640 by the linear displacement member 680 (e.g., solenoid, linear actuator, or reciprocating motor, cam, rotating reciprocating motor, etc.), the flexible inlet tubing 610 is in a non-compressed shape or state as shown in Figure 6B allowing unrestricted passage therethrough of the vacuum pressure. Whereas, Figure 6C depicts the compression plate 640 advanced by the linear displacement mechanism 680 towards the stationary base 640' at least partially compressing or constricting the flexible inlet tubing 610 therebetween reducing the volume and displacing the fluid collected therein thereby generating a positive pressure pulse (i.e., injection of positive pressure). Since the flexible inlet tubing 610 is secured respectively thereto, during subsequent retraction of the compression plate 640 relative to the stationary base 640' return to the non-compressed state or shape is forcibly assisted (rather than the flexible inlet tubing 610 recovering naturally (i.e., unforced or unassisted) on its own) thereby minimizing recovery or restoration time while maximizing cycling frequency (Figure 6D). In addition, gripping of the flexible inlet tubing 610 to the respective compression plate 640 and stationary base 640' also resists or prevents collapse while under vacuum.

Figures 7A and 7B depict still another example of a positive pressure pulse mechanism disposed along a section of flexible inlet tubing and a separate independently displaceable gating mechanism disposed proximally thereto acting as a valve controlling (i.e., restricting, although not necessarily cutting off) passage therethrough of the vacuum pressure generated by the vacuum pump 105. In this example, the positive pressure pulse mechanism includes a first linear displacement mechanism 788 (e.g., linear actuator, solenoid, reciprocating motor, cam, rotating reciprocating motor, etc.) 788 for intermittently cyclically advancing or retracting a plunger 740 connected thereto. While the plunger 740 is in the advanced state the flexible inlet tubing 110 (i.e., vacuum line) is at least partially, albeit not necessarily fully or completely, compressed reducing the volume and displacing the fluid collected therein thereby generating the positive pressure pulse (i.e., injection of positive pressure) negating the vacuum pressure. Proximally of the plunger 790 is the separate displaceable gating mechanism (e.g., a pin 790) independently displaceable via a second linear displacement mechanism 786 (e.g., linear actuator, solenoid, reciprocating motor, cam, rotating reciprocating motor, etc.) controllable independently of the first linear displacement mechanism 788. Pin 790 acts as a valve (e.g., pinch valve) controlling (e.g., restricting, albeit not necessarily closing off) passage therethrough of the vacuum pressure generated by the vacuum pump 105. Whereas, the first solenoid 788 controls the extent of compressive force imposed on the flexible inlet tubing 110 by varying the extent of advancement of the plunger 740. When advanced by the first linear displacement mechanism 788 the plunger 790 at least partially compresses, not necessarily flattening, the flexible inlet tubing 110 reducing the volume and displacing the fluid collected therein thereby generating the positive pressure pulse (i.e., injection of positive pressure). With the gating mechanism 786 in the fully closed position as illustrated in the example of Figure 7B, the efficiency of the plunger 740 is maximized since the displaced fluid travels exclusively distally towards the aspiration catheter without any loss proximally to the vacuum pump. Figure 7A depicts the vacuum pressure interval of the cyclic aspiration pressure waveform in which both the pin 790 and the plunger 740 are retracted with the flexible inlet tubing 110 in a non-compressed state allowing unrestricted passage therethrough of the vacuum pressure generated by the vacuum pump 705. While, in Figure 7B the pin 790 is advanced by the second linear displacement mechanism 786 at least partially restricting, possibly cutting off completely, passage therethrough of the vacuum pressure generated by the vacuum pump 105. Simultaneously therewith the plunger 740 advanced independently by the first linear displacement mechanism 788 at least partially compresses, not necessarily completely flattening, the flexible inlet tubing 110 reducing the volume and displacing the fluid collected therein thereby generating the positive pressure pulse (i.e., injection of positive pressure). It is noted that the pin 790 when advanced need not necessarily occlude, block, or completely cut off passage therethrough the flexible inlet tubing 110 (i.e., vacuum line) of the vacuum pressure. If the positive pressure injected is sufficient to negate the vacuum pressure and provide a positive pressure pulse towards the clot then it is contemplated that the flexible inlet tubing 110 (i.e., vacuum line) may remain partially (e.g., pin 790 in a partially advanced state) or completely open (e.g., pin 790 in a fully retracted state). Control of the extent of advancement of the plunger 740 by the first linear displacement mechanism 788 and hence extent of compression or constriction of the flexible inlet tubing 110 may be used to vary or adjust the amplitude of the positive pressure pulse generated.

The example in Figure 7A & 7B employs two independently controlled linear displacement mechanisms (e.g., solenoids 786, 788) for independently controlling/displacing two separate components (e.g., pin 790 acting as a valve controlling passage therethrough of the vacuum pressure generated by the vacuum pump 705 and plunger 740 compressing the flexible inlet tubing 110 reducing the volume and displacing the fluid collected therein thereby generating the positive pressure pulse (i.e., injected positive pressure). Rather than employing two separate components each having their own associated linear displacement mechanism, Figures 8A & 8B is an alternative example of a single linear displacement mechanism 888 (e.g., linear actuator, solenoid, reciprocating motor, cam, rotating reciprocating motor, etc.) controlling a single component having two heads including a spring-loaded pin head 890 disposed proximally (i.e., towards the vacuum pump) in an axial direction of a plunger head 840. When advanced the spring-loaded pin head 890 controls passage therethrough of the vacuum pressure generated by the vacuum pump 805, while the plunger head 840 at least partially compresses, possibly flattening, the flexible inlet tubing 110 reducing the volume and displacing the fluid collected therein thereby generating the positive pressure pulse (i.e., injection of positive pressure). Once again it is noted that the pin head 890 when advanced need not necessarily occlude, block, or completely cut off passage therethrough the flexible inlet tubing 110 (i.e., vacuum line) of the vacuum pressure. With the pin head 890 in the fully closed position, the efficiency of the plunger head 840 is maximized since the displaced fluid travels exclusively distally towards the aspiration catheter without any loss proximally to the vacuum pump. If the positive pressure injected is sufficient to negate the vacuum pressure and provide a positive pressure pulse towards the clot then the flexible inlet tubing 110 (i.e., vacuum line) may remain partially or completely open (i.e., pin head 890 in a partially advanced or fully retracted state). Control of the extent of advancement of the plunger head 840 by the single linear displacement mechanism 888 and hence extent of compression or constriction of the inlet tubing 110 may be used to vary or adjust the amplitude of the positive pressure pulse generated.

The example cyclic aspiration system in Figure 9 differs from the previous example shown in Figure 7A & 7B in that two rotating reciprocating motors are employed to independently control the pin (displaceable gating device) and plunger (positive pressure pulse generator mechanism) instead of a linear actuator. Referring to Figure 9, a reciprocating plunger 940 is cyclically actuated (i.e., advanced and retracted) by a first rotating to linear motion mechanism including by way of illustrative example a cam 912, an inner drive gear 913 connected to a motor, and a roller bearing 914. The reciprocating plunger 940 preferably is spring-loaded 915 within a guide block 920 providing axial resistance restoring the plunger 940 to a retracted state when not advanced by the cam 912. The reciprocating plunger 940 is depicted in Figure 9 in an advanced state at least partially compressing, possibly flattening, the flexible inlet tubing 110 (i.e., vacuum line) reducing the volume displacing the fluid therein thereby generating the positive pressure pulse (i.e., injection of positive pressure) negating the vacuum pressure. Following the previous examples in Figures 7A, 7B, 8A & 8B, the reciprocating plunger 940 is illustrated in Figure 9 with a separate reciprocating pin 990 (acting as a displaceable gating mechanism) independently actuatable via an associated rotating to linear motion mechanism including by way of illustrative example a gear 913, a cam 912, an inner drive gear 913 connected to a motor, and roller bearing 914 similar to that of the reciprocating plunger 940. The reciprocating pin 990 serving as a valve to control (i.e., at least restrict, albeit not necessarily close off) passage therethrough of the vacuum pressure generated by the vacuum pump 105 (Figure 9). Following the example in Figure 8A & 8B described above, it is further contemplated that a single rotating to linear motion mechanism by way of illustrative example including a gear 913, a cam 912, an inner drive gear 913 connected to a motor, and roller bearing 914 may control a single component having two reciprocating heads (i.e., a plunger head 940 and a pin head 990 arranged proximally thereto). The particular rotating to linear motion mechanism used may be modified as desired and, in the case of two separate rotating to linear motion mechanisms to control two separate components (e.g., plunger and pin), need not necessarily by the same arrangement for both.

As previously mentioned, the amplitude of the positive pressure pulse generated using any one of the positive pressure pulse generator mechanisms described above may be varied or adjusted by changing the extent of compression of the flexible inlet tubing (i.e., vacuum line) (e.g., the extent of advancement of the plunger). In this regard, such adjustment of the amplitude of the positive pressure pulse may be based on a detected or monitored pressure by a pressure sensor associated with the flexible inlet tubing (i.e., vacuum line) disposed proximally of the proximal hub of the aspiration catheter. Based on such detected pressure, the amplitude of the positive pressure pulse produced in the flexible inlet tubing (i.e., vacuum line) using any one of the previously described exemplary positive pressure pulse mechanisms may be adjusted or controlled by varying the extent of compression of the flexible inlet tubing (e.g., extent of advancement of the plunger). In this regard, it is further recognized that the extent of sealing by the clot captured at the distal tip/end of the aspiration catheter is indicative of the type of clot (e.g., firm clot is fibrin dominant/rich, tough, and less likely to fragment vs. friable clot is dominant/rich in red blood cells, soft, and fragments easily). By way of example, as measured histologically by area firm clots have a red blood cell content in a range of approximately 0 to approximately 20%. When subject to aspiration firm clots form a tighter seal when captured at the distal tip/end of the aspiration catheter resulting in a substantially consistent repeating or regular cyclic pressure waveform measured at the distal tip/end and hence at the proximal hub as detected by the pressure sensor. Figure 10B is a graphical representation of a representative example of a substantially consistently repeatable cyclic pressure waveform indicative of a firm clot. In contrast to a firm clot, a friable clot fragments easily creating a more erratic, inconsistent, non-repeating, irregular cyclic aspiration pressure waveform due to the clot fragments entering the aspiration catheter and/or intermittently sealing at the distal tip/end. A representative example of an erratic, inconsistent, non-repeating, irregular cyclic aspiration pressure waveform indicative of a friable clot is illustrated in Figure 10C. Figure 10A is an exemplary aspiration catheter 1035 delivered through the vessel depicting a clot captured at the distal tip/end. Inlet tubing 1010 connects in fluid communication the proximal hub 1020 of the aspiration catheter 1035 to a pulsatile vacuum pump system 1005 producing a cyclic aspiration pressure waveform of intermittent cyclic intervals of vacuum pressure (i.e., below atmospheric pressure) and positive pressure (i.e., higher than vacuum pressure, possibly higher than atmospheric pressure). For example, the pulsatile vacuum pump system 1005 may be a vacuum pump and any one of the example positive pressure pulse generator mechanisms illustrated herein and described above to produce the cyclic aspiration pressure waveform by intermittently cyclically compressing a section along the flexible inlet tubing 1010. Disposed proximally of the proximal hub 1020 is a pressure sensor(s) 1030 for monitoring a cyclic aspiration pressure waveform over time. Data of the pressure detected by the pressure sensor(s) 1030 and/or via a user interface is received as input to a controller 1050 (e.g., processor) for adjustment & optimization of one or more cyclic aspiration pressure waveform parameters (e.g., amplitude of vacuum pressure (i.e., minimum pressure); amplitude of positive pressure pulse (i.e., peak pressure); and/or cycling frequency) associated with the pulsatile vacuum pump system 1005 based on the type of clot (e.g., firm vs. friable) as determined by the monitored cyclic aspiration pressure waveform (e.g., substantially consistently repeatable or non-repeating) detected by the pressure sensor(s) 1030.

The descriptions contained herein are examples. As described herein, the present disclosure contemplates many variations and modifications of a cyclic aspiration system for producing a cyclic aspiration pressure waveform including intermittent cyclic intervals of vacuum pressure (i.e., below atmospheric pressure) and positive pressure (i.e., higher than vacuum pressure, possibly higher than atmospheric pressure) using a vacuum pump connected in fluid communication with the hub of an aspiration catheter and a positive pressure pulse generator intermittently cyclically externally compressing a section along the flexible inlet tubing reducing the volume and displacing fluid collected therein thereby generating a positive pressure pulse (i.e., injection of positive pressure). The positive pressure pulse generator mechanism in accordance with the present disclosure advantageously is arranged externally of the flexible inlet tubing (i.e., vacuum line), not contaminated by blood, and therefore reusable; whereas the flexible inlet tubing is inexpensive, contaminated by blood, and discardable after a single use or procedure thereby preventing clogging. Modifications and variations are apparent to those having skilled in the pertinent art according to the teachings of this disclosure.

## Claims

1. A cyclic aspiration system for the capture and removal of occlusions or clots in a vessel, the system producing a cyclic aspiration pressure waveform of intermittent cyclic intervals of vacuum pressure below atmospheric pressure and positive pressure higher than vacuum pressure, and the system comprising:
a vacuum pump (105) generating the vacuum pressure;
a flexible inlet tubing (110, 510, 610) having a proximal end, an opposite distal end; the proximal end of the flexible inlet tubing is connected in fluid communication to the vacuum pump;
an aspiration catheter (135) having a distal tip and a proximal hub (120) connected in fluid communication to the distal end of the flexible inlet tubing; and
a positive pressure pulse generator mechanism (140, 240, 440, 540, 540', 640, 640', 740, 840, 940) intermittently cyclically applying an external force compressing a section of the flexible inlet tubing reducing internal volume and displacing fluid collectable therein thereby generating a positive pressure pulse; wherein upon withdrawal of the external force applied by the positive pulse generator mechanism, the flexible inlet tubing being configured to be forcibly restorable to a non-compressed state increasing the internal volume while reducing pressure therein until eventual regeneration of the vacuum pressure thereby minimizing recovery time and maximizing cycling frequency.

2. The cyclic aspiration system in accordance with claim 1, wherein the positive pressure pulse generator mechanism is arranged externally of the flexible inlet tubing, not contaminatable by blood and reusable; whereas the flexible inlet tubing is contaminatable by blood, and discardable after a single use.

3. The cyclic aspiration system in accordance with claim 2, wherein the positive pressure pulse generator mechanism is a displaceable plunger (140, 740, 840), a pressurizable bladder (240), a rotatable arm (440), a pair of electromagnets (540, 540'), or a compression plate (640).

4. The cyclic aspiration system in accordance with claim 1, wherein the flexible inlet tubing is forcibly restorative to the non-compressed state via a radially self-expanding restoring structure (115) disposed therein coinciding with the section compressed by the external force applied by the positive pressure pulse generator mechanism; and the radially self-expanding restoring structure also providing resistance against collapse of the flexible inlet tubing while under the vacuum pressure.

5. The cyclic aspiration system in accordance with claim 1, wherein the flexible inlet tubing is forcibly restorative to the non-compressed state by being held in place via a retaining member while subjected to an external restoring force imposed by the positive pressure pulse generator mechanism when the externally applied force is withdrawn; and the retaining member resisting collapse of the flexible inlet tubing while under the vacuum pressure.

6. The cyclic aspiration system in accordance with claim 5, wherein the flexible inlet tubing is electrically conductive and the positive pressure pulse generator mechanism comprises a first electromagnet (540) having a concave contacting surface and a second electromagnet (540') having a planar contacting surface with the flexible inlet tubing disposed therebetween; when the second electromagnet is energized the flexible inlet tubing being compressed while being drawn to the planar contacting surface; and wherein the retaining member is a permanent magnet (560) associated with the second electromagnet maintaining the flexible inlet tubing in place while simultaneously being drawn to the concave contacting surface of the first electromagnet when energized.

7. The cyclic aspiration system in accordance with claim 5, wherein the flexible inlet tubing is forcibly restorative to the non-compressed state by being mechanical securable between a stationary base (640') and a linearly displaceable member (640) moveable relative thereto.

8. The cyclic aspiration system in accordance with claim 7, wherein the flexible inlet tubing is restorative to the non-compressed state via radial resistance exhibited by the flexible inlet tubing having a non-circular shape that is extruded, cast, or molded; and the non-circular shape also providing resistance against collapse of the flexible inlet tubing while under the vacuum pressure.

9. The cyclic aspiration system in accordance with claim 1, further comprising a displaceable gating device (740, 940) associated with the flexible inlet tubing disposed between the positive pressure pulse generator mechanism and the vacuum pump; the displaceable gating device controlling passage therethrough of the vacuum pressure generated by the vacuum pump; wherein the positive pressure pulse generator mechanism and the displaceable gating device are separate components independent of one another or a single integrated component.

## Patentansprüche

1. Zyklisches Aspirationssystem für das Einfangen und Entfernen von Okklusionen oder Gerinnseln in einem Gefäß, wobei das System eine zyklische Aspirationsdruckwellenform von intermittierenden zyklischen Intervallen von Vakuumdruck unterhalb eines atmosphärischen Drucks und Überdruck, der höher als der Vakuumdruck ist, produziert, und das System umfassend:
eine Vakuumpumpe (105), die den Vakuumdruck erzeugt;
einen flexiblen Einlassschlauch (110, 510, 610), der ein proximales Ende, ein gegenüberliegendes distales Ende aufweist; wobei das proximale Ende des flexiblen Einlassschlauchs in Fluidkommunikation mit der Vakuumpumpe verbunden ist;
einen Aspirationskatheter (135), der eine distale Spitze und eine proximale Nabe (120) aufweist, die in Fluidkommunikation mit dem distalen Ende des flexiblen Einlassschlauchs verbunden ist; und
einen Überdruckimpulserzeugermechanismus (140, 240, 440, 540, 540', 640, 640', 740, 840, 940), der eine externe Kraft intermittierend zyklisch ausübt, die einen Abschnitt des flexiblen Einlassschlauchs komprimiert, wobei ein Innenvolumen reduziert und darin ansammelbares Fluid verschoben wird, wobei dadurch ein Überdruckimpuls erzeugt wird; wobei bei Aufhebung der durch den Überdruckimpulserzeugermechanismus ausgeübten externen Kraft der flexible Einlassschlauch konfiguriert ist, um in einen nicht-komprimierten Zustand zwangsweise rückführbar zu sein, wobei das Innenvolumen erhöht wird, während der Druck darin bis zu einer letztendlichen Regeneration des Vakuumdrucks reduziert wird, wobei dadurch eine Wiederherstellungszeit minimiert und eine Zyklusfrequenz maximiert wird.

2. Zyklisches Aspirationssystem nach Anspruch 1, wobei der Überdruckimpulserzeugermechanismus außerhalb des flexiblen Einlassschlauchs angeordnet, nicht durch Blut kontaminierbar und wiederverwendbar ist; wohingegen der flexible Einlassschlauch durch Blut kontaminierbar ist und nach einer einmaligen Verwendung entsorgbar ist.

3. Zyklisches Aspirationssystem nach Anspruch 2, wobei der Überdruckimpulserzeugermechanismus ein verschiebbarer Kolben (140, 740, 840), eine druckbeaufschlagbare Blase (240), ein drehbarer Arm (440), ein Paar Elektromagnete (540, 540') oder eine Kompressionsplatte (640) ist.

4. Zyklisches Aspirationssystem nach Anspruch 1, wobei der flexible Einlassschlauch über eine darin eingerichtete radial selbstexpandierende Rückführstruktur (115) in den nicht-komprimierten Zustand zwangsweise rückführbar ist, die mit dem Abschnitt übereinstimmt, der durch die durch den Überdruckimpulserzeugermechanismus ausgeübte externe Kraft komprimiert wird; und die radial selbstexpandierende Rückführstruktur auch einen Widerstand gegen ein Kollabieren des flexiblen Einlassschlauchs bereitstellt, während er unter dem Vakuumdruck steht.

5. Zyklisches Aspirationssystem nach Anspruch 1, wobei der flexible Einlassschlauch dadurch in den nicht-komprimierten Zustand zwangsweise rückführbar ist, dass er über ein Rückhalteelement in seiner Position festgehalten wird, während er einer externen Rückführkraft ausgesetzt ist, die durch den Überdruckimpulserzeugermechanismus aufgebracht wird, wenn die extern ausgeübte Kraft aufgehoben ist; und das Rückhalteelement dem Kollabieren des flexiblen Einlassschlauchs widersteht, während er unter dem Vakuumdruck steht.

6. Zyklisches Aspirationssystem nach Anspruch 5, wobei der flexible Einlassschlauch elektrisch leitfähig ist und der Überdruckimpulserzeugermechanismus einen ersten Elektromagneten (540), der eine konkave kontaktierende Oberfläche aufweist, und einen zweiten Elektromagneten (540'), der eine planare kontaktierende Oberfläche aufweist, umfasst, wobei der flexible Einlassschlauch dazwischen eingerichtet ist; wobei, wenn der zweite Elektromagnet erregt ist, der flexible Einlassschlauch komprimiert wird, während er zu der planaren kontaktierenden Oberfläche gezogen wird; und wobei das Rückhalteelement ein Dauermagnet (560) ist, der dem zweiten Elektromagneten zugeordnet ist, der den flexiblen Einlassschlauch in seiner Position hält, während er gleichzeitig zu der konkaven kontaktierenden Oberfläche des ersten Elektromagneten gezogen wird, wenn dieser erregt ist.

7. Zyklisches Aspirationssystem nach Anspruch 5, wobei der flexible Einlassschlauch dadurch in den nicht-komprimierten Zustand zwangsweise rückführbar ist, dass er zwischen einer stationären Basis (640') und einem relativ dazu bewegbaren linear verschiebbaren Element (640) mechanisch befestigbar ist.

8. Zyklisches Aspirationssystem nach Anspruch 7, wobei der flexible Einlassschlauch in den nicht-komprimierten Zustand über einen radialen Widerstand rückführbar ist, der durch den flexiblen Einlassschlauch vorgewiesen wird, der eine nicht-kreisförmige Form aufweist, die extrudiert, gegossen oder geformt ist; und die nicht-kreisförmige Form auch einen Widerstand gegen das Kollabieren des flexiblen Einlassschlauchs bereitstellt, während er unter dem Vakuumdruck steht.

9. Zyklisches Aspirationssystem nach Anspruch 1, ferner umfassend eine dem flexiblen Einlassschlauch zugeordnete verschiebbare Absperrvorrichtung (740, 940), die zwischen dem Überdruckimpulserzeugermechanismus und der Vakuumpumpe eingerichtet ist; wobei die verschiebbare Absperrvorrichtung den Durchgang des durch die Vakuumpumpe erzeugten Vakuumdrucks dahindurch steuert; wobei der Überdruckimpulserzeugermechanismus und die verschiebbare Absperrvorrichtung getrennte Komponenten, die voneinander unabhängig sind, oder eine einzige integrierte Komponente sind.

## Revendications

1. Système d'aspiration cyclique destiné à la capture et à l'élimination d'occlusions ou de caillots dans un vaisseau, le système produisant une forme d'onde de pression d'aspiration cyclique d'intervalles cycliques intermittents de pression de vide sous la pression atmosphérique et de pression positive supérieure à la pression de vide, et le système comprenant :
une pompe à vide (105) générant la pression de vide ;
un tubage d'entrée flexible (110, 510, 610) ayant une extrémité proximale, une extrémité distale opposée ; l'extrémité proximale du tubage d'entrée flexible est raccordée en communication fluidique à la pompe à vide ;
un cathéter d'aspiration (135) ayant une pointe distale et une embase proximale (120) raccordée en communication fluidique à l'extrémité distale du tubage d'entrée flexible ; et
un mécanisme générateur d'impulsions de pression positive (140, 240, 440, 540, 540', 640, 640', 740, 840, 940) appliquant cycliquement de façon intermittente une force externe comprimant une section du tubage d'entrée flexible en réduisant le volume interne et en déplaçant un fluide pouvant être collecté dans celui-ci en générant de ce fait une impulsion de pression positive ; dans lequel lors du retrait de la force externe appliquée par le mécanisme générateur d'impulsions positives, le tubage d'entrée flexible est conçu pour pouvoir se rétablir de force à un état non comprimé en augmentant le volume interne tout en réduisant la pression dans celui-ci jusqu'à une éventuelle régénération de la pression de vide en minimisant de ce fait le temps de rétablissement et en maximisant la fréquence de cyclage.

2. Système d'aspiration cyclique selon la revendication 1, dans lequel le mécanisme générateur d'impulsions de pression positive est agencé à l'extérieur du tubage d'entrée flexible, ne pouvant pas être contaminé par du sang et étant réutilisable ; alors que le tubage d'entrée flexible peut être contaminé par du sang et est jetable après un usage unique.

3. Système d'aspiration cyclique selon la revendication 2, dans lequel le mécanisme générateur d'impulsions de pression positive est un piston déplaçable (140, 740, 840), une vessie pressurisable (240), un bras rotatif (440), une paire d'électroaimants (540, 540'), ou une plaque de compression (640).

4. Système d'aspiration cyclique selon la revendication 1, dans lequel le tubage d'entrée flexible peut se rétablir de force à l'état non comprimé par l'intermédiaire d'une structure de rétablissement radialement auto-expansible (115) disposée dans celui-ci coïncidant avec la section comprimée par la force externe appliquée par le mécanisme générateur d'impulsions de pression positive ; et la structure de rétablissement radialement auto-expansible fournissant également une résistance vis-à-vis d'un écrasement du tubage d'entrée flexible alors qu'il est sous la pression de vide.

5. Système d'aspiration cyclique selon la revendication 1, dans lequel le tubage d'entrée flexible peut se rétablir de force à l'état non comprimé en étant maintenu en place par l'intermédiaire d'un élément de retenue alors qu'il est soumis à une force de rétablissement externe exercée par le mécanisme générateur d'impulsions de pression positive lorsque la force appliquée de façon externe est retirée ; et l'élément de retenue résiste à un écrasement du tubage d'entrée flexible alors qu'il est sous la pression de vide.

6. Système d'aspiration cyclique selon la revendication 5, dans lequel le tubage d'entrée flexible est électroconducteur et le mécanisme générateur d'impulsions de pression positive comprend un premier électroaimant (540) ayant une surface de contact concave et un second électroaimant (540') ayant une surface de contact plane avec le tubage d'entrée flexible disposé entre eux ; lorsque le second électroaimant est alimenté en énergie le tubage d'entrée flexible est comprimé tout en étant tiré vers la surface de contact plane ; et dans lequel l'élément de retenue est un aimant permanent (560) associé au second électroaimant maintenant le tubage d'entrée flexible en place tout en étant simultanément tiré vers la surface de contact concave du premier électroaimant lorsqu'il est alimenté en énergie.

7. Système d'aspiration cyclique selon la revendication 5, dans lequel le tubage d'entrée flexible peut se rétablir de force à l'état non comprimé en pouvant être fixé mécaniquement entre une base stationnaire (640') et un élément déplaçable linéairement (640) mobile par rapport à celle-ci.

8. Système d'aspiration cyclique selon la revendication 7, dans lequel le tubage d'entrée flexible peut se rétablir à l'état non comprimé par l'intermédiaire d'une résistance radiale présentée par le tubage d'entrée flexible ayant une forme non circulaire qui est extrudé, coulé ou moulé ; et la forme non circulaire fournissant également une résistance vis-à-vis de l'écrasement du tubage d'entrée flexible alors qu'il est sous la pression de vide.

9. Système d'aspiration cyclique selon la revendication 1, comprenant en outre un dispositif d'obturation déplaçable (740, 940) associé au tubage d'entrée flexible disposé entre le mécanisme générateur d'impulsions de pression positive et la pompe à vide ; le dispositif d'obturation déplaçable commandant le passage à travers celui-ci de la pression de vide générée par la pompe à vide ; dans lequel le mécanisme générateur d'impulsions de pression positive et le dispositif d'obturation déplaçable sont des composants séparés indépendants l'un de l'autre ou un composant d'un seul tenant.
